# EUROPEAN PATENT SPECIFICATION

(11) **EP 2 409 690 B1**
(45) Date of publication and mention of the grant of the patent: **09.11.2022**
(21) Application number: 11175793.6
(22) Date of filing: 21.09.2004
(51) Int. Cl.: A61K 9/20, A61K 9/28, A61K 31/565, A61K 31/567

(54) **Improved stability of progestogen formulations**
Verbesserte Stabilität von Progestogenformulierungen
Stabilité améliorée de formulations de progestogène

(30) Priority: 29.09.2003 DK 200301408; 09.10.2003 US 509962 P
(43) Date of publication of application: 25.01.2012
(62) Divisional of application: 04762859.9
(73) Proprietor: Novo Nordisk Health Care AG, The Circle 32/38 8058 Zürich-Flughafen (CH)
(72) Inventor: FRIIS, Vibeke, 2880 Bagsværd (DK)
(74) Representative: Mikkelsen, Per Jensen

(56) References cited:
- US-A- 4 826 831
- EDITIO CANTOR VERLAG ED - ROTE LISTE SERVICE GMBH (ED): "ROTE LISTE 2000, PASSAGE", 1 January 2000 (2000-01-01), ROTE LISTE 2000. ARZNEIMITTELVERZEICHNIS FUER DEUTSCHLAND (EINSCHLIESLICH EU - ZULASSUNGEN UND BESTIMMTER MEDIZINPRODUKTE); [ROTE LISTE], AULENDORF : EDITIO CANTOR, DE, PAGE(S) 76075, XP002676918, ISBN: 978-3-87193-218-2 * paragraph [76075] *
- "VIDAL", 1997, EDITIONS DU VIDAL, PARIS, XP002316912, * page 1699 - page 1700 *
- "VIDAL", 1997, EDITIONS DU VIDAL, PARIS, XP002316913, * page 903 - page 904 *
- EDITIO CANTOR VERLAG ED - ROTE LISTE SERVICE GMBH (ED): "ROTE LISTE 2002, PASSAGE", 1 January 2002 (2002-01-01), ROTE LISTE 2002. ARZNEIMITTELVERZEICHNIS FUER DEUTSCHLAND (EINSCHLIESLICH EU - ZULASSUNGEN UND BESTIMMTER MEDIZINPRODUKTE); [ROTE LISTE], AULENDORF : EDITIO CANTOR, DE, PAGE(S) 76110, XP002316915, ISBN: 978-3-87193-252-6 * paragraph [76110] *

## Description

### Field of this Invention

The present invention relates to progestogen-containing pharmaceutical formulations.

### Background of this Invention

Progestogens have wide use as therapeutic agents, such as, for example, in hormone replacement therapy, and as components of contraceptives and for the treatment of osteoporosis. Nonetheless, with an increasing awareness of the risks involved with various types of combined estrogen/progestogen administration, there have been increased efforts to formulate products that deliver lower dosages of these hormones. Unfortunately, lowering the concentration of a steroid component in a formulation may result in some degree of lowered stability and/or recoverability of the component.

Examples of documents defining the general state of the art are the following patents and patent applications: EP 337,938 A2; EP 787,488 A1; EP 1,334,724 A1; US 5,770,227; US 6,284,263 B1; US 2002/0168412 A1; and US 2003/0077297 A1.

The Activelle^{®} film-coated tablet composition is disclosed on page 76075 of the book "Rote Liste 2000, Arzneimittelverzeichnis fuer Deutschland".

Thus, there is a need in the art for improved formulations containing progestogens that exhibit acceptable stability of the progestogen component.

Hence, one object of this invention is to provide a formulation containing progestogen in low amount which has a satisfactory high stability.

### Summary of this Invention

The present invention provides a pharmaceutical tablet comprising (i) norethindrone acetate (NETA) in the range from 0.05 to 0.35 mg per unit dosage form or the corresponding amount of norethindrone (NET) or a corresponding amount of a soluble ester or a salt of NET, and (ii) a cellulosic binder in the range from 2 to 10% w/w, and (iii) a cellulosic film-coating in the range 1.5 to 5% w/w.

The present disclosure provides pharmaceutical formulations comprising (i) a progestogen and (ii) a cellulosic binder, comprising not more than about 1.5% (weight/weight (hereinafter designated w/w)) norethisterone acetate (hereinafter designated NETA) relative to the total formulation, such as, for example, from about 0.05% to about 1.5% (w/w) NETA or the equivalent amount of a non-NETA progestogen. In different embodiments, the formulation may comprise less than about 0.75%, about 0.5%, about 0.25%, or about 0.1% (w/w) NETA relative to the total formulation, such as, for example, between about 0.05-0.75%; 0.05-0.5%; 0.05-0.25%; 0.05-1.5%; 0.1-0.75%; 0.1-0.5%; 0.1-0.25%; 0.25-0.75%; 0.25-0.5%, NETA or the equivalent amount of a non-NETA progestogen. In another aspect, the disclosure provides pharmaceutical formulations comprising (i) a progestogen and (ii) a cellulosic binder, comprising not more than 0.35 mg NETA per unit dosage form, including, without limitation, not more than 0.3, 0.25, 0.2, 0.1, or 0.05 mg NETA per unit dosage form, such as, for example, between about 0.05-0.3 mg; 0.05-0.25 mg; 0.05-0.1 mg; 0.1-0.3 mg; 0.1-0.25 mg; and 0.1-0.2 mg NETA or the equivalent amount of a non-NETA progestogen. In these embodiments, typically, the unit dosage form is a tablet having a total weight (excluding coating) of between about 25 and 125 mg.

Preferably, the stability of the progestogen component is enhanced relative to the stability of progestogen in a parallel formulation in which the non-cellulosic binder is replaced with an equivalent amount of a cellulosic binder. In one series of embodiments, the stability of the progestogen component is enhanced relative to the stability of progestogen in a parallel formulation in which the polyvinylpyrrolidone is replaced with an equivalent amount of hydroxypropylcelllulose. In another series of embodiments, the stability of the progestogen component is enhanced relative to the stability of progestogen in a parallel formulation in which the gelatin is replaced with an equivalent amount of hydoxypropylcellulose.

Non-limiting examples of suitable cellulosic binders for use in the present invention include methylcellulose, sodium carboxymethylcellulose (for example Tylose^{™}), ethylcellulose (for example Ethocel^{™}), hydroxypropyl methyl cellulose (HPMC) (Shin-Etsu Chemical Co., Ltd.), and hydroxypropyl cellulose (for example Klucel^{™}).

In some embodiments, the formulation has the composition stated in table I below:

**Table I**

| | |
|---|---|
| Estradiol hemihydrate | about 0.5 mg |
| Norethisterone Acetate | about 0.1 mg |
| Lactose monohydrate | about 37 mg |
| Maize starch | about 37 mg |
| Hydroxypropyl cellulose or hydroxypropyl methyl cellulose | about 3 mg |
| Talc | about 0.8 mg |
| Magnesium stearate | about 0.4 mg |

In another aspect, the disclosure provides methods for stabilizing a progestogen component of a pharmaceutical formulation, particularly a formulation in which NETA is present at a concentration not more than about 1.5% w/w relative to the total formulation (such as, for example, between about 0.05-1.5% w/w) or a formulation in which NETA is present in an amount not more than about 0.35 mg per unit dosage form or the equivalent amount of a non-NETA progestogen. The methods are carried out by contacting the progestogen with a stabilizing-effective amount of a cellulosic binder. A stabilizing-effective amount is an amount of binder that enhances the shelf-life of the progestogen component. The stability of a formulation according to this invention can be determined by comparing the stability of progestin in said formulation with the stability of progestin in an identical formulation which deviates only in that the cellulosic binder has been exchanged by an identical amount of polyvinylpyrrolidone. Conveniently, such tests are performed at a temperature of 40°C and a relative humidity of 75% and analyses for stability are made after 0, 3, and 6 months. Conveniently, the stability is enhanced by about 5% or more.

In another aspect, the invention provides methods for treating a progestogen-responsive syndrome, which are carried out by administering to a patient in need of such treatment an effective amount for treating the syndrome of a formulation of the invention.

### Detailed Description of the Invention

The present invention relates to improved formulations of progestogen-containing medications. Such formulations can, for example, be administered orally or vaginally. The present inventor has found that the use of one or more cellulosic binders in progestogen-containing formulations provides superior stability and/or recoverability of the active ingredient relative to conventional formulations, particularly when the formulation contains NETA at a concentration not more than about 1.5% w/w relative to the total weight of the composition, such as, for example, not more than about 0.35 mg NETA or the equivalent amount of a non-NETA progestogen in a conventional unit dosage form such as a tablet. In some embodiments, the content of NETA in the formulation is not more than about 0.75%, 0.5%, 0.25%, or 0.1% w/w relative to the total formulation, such as, for example, between about 0.05-0.75%, 0.05-0.5%, 0.05-0.25%, 0.05-1.5%; 0.1-0.75%; 0.1-0.5%; 0.1-0.25%; 0.25-0.75%; 0.25-0.5% w/w NETA or the equivalent amount of a non-NETA progestogen. In some embodiments, the content of NETA in the formulation is less than about 0.35 mg/unit dosage form, including, without limitation, not more than about 0.3, 0.25, 0.2, and 0.1 mg NETA/unit dosage form, such as, for example, between about 0.05-0.3 mg; 0.05-0.25 mg; 0.05-0.1 mg; 0.1-0.3 mg; 0.1-0.25 mg; and 0.1-0.2 mg NETA per unit dosage form or the equivalent amount of a non-NETA progestogen.

Stability and/or recoverability as used herein refers to the ability to detect the progestogen active ingredient in a formulation, typically by HPLC, after a storage interval under predetermined conditions of temperature and humidity (see, for example, Example 1 below.)

Progestogen as used herein includes any drug that binds to the progestogen receptor and induces a progestational effect. Progestogens include, without limitation, all of the known progestogens and derivatives of progesterone or testosterone that have progestogen activity. The known synthetic progestogens are mainly derivatives of 17α-hydroxyprogesterone or 19-nortestosterone. These progestogens can be classified into three groups: the pregnane, estrane, and gonane derivatives. The pregnane progestogens, derived from 17α-hydroxyprogesterone, include, for example, medroxyprogesterone acetate, chlormadinone acetate, megestrol acetate, and cyproterone acetate. The estranes, derived from 19-nortestosterone include norethindrone, norethynodrel, lynestrenol, norethindrone acetate (NETA), ethynodiol acetate, and norethindrone enanthate. The gonanes are derived from the basic estrane structure, with the addition of an ethyl group of position 13 of the molecule. Drugs in this group include, for example, norgestrel (-dextro and -laevo), norgestimate, desogestrel, and gestodene. Additional non-limiting examples of progestogens include osaterone, dienogest, chlormadinone acetate, and drospirenone.

One way of determining which amount of a non-NETA progestogen is equivalent with NETA is to determine the amount which gives the same action on the endometrium. This could be designated an endometrium transformation dosage. Assessment of the potency of orally administered progesterones in women can be made as described in *Fertility and Sterility* 46 (1986), 1062 *et seq*.). Examples of endometrium transformation dosages are 1 mg NETA = 5 mg medroxyprogestogen acetate and 1 mg NETA = 1 mg cyproterone acetate. Other figures are known from the literature.

Progestogens may be formulated as pharmaceutically acceptable salts, including, without limitation, organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including, without limitation, hydrochloride, sulfate, nitrate, bicarbonate, and carbonate salts.

In a preferred embodiment of this invention, the progestogen is a 19-nortestosterone derivative, preferably norgestrel, laevonorgestrel, NET or NETA.

In another preferred embodiment of this invention, the progestogen is a soluble ester or salt of norethindrone, preferably a soluble ester, more preferred NETA or norethindrone enanthate. In a further preferred embodiment of this invention, the progestogen is NETA.

In practicing the present invention, a progestogen-containing formulation is prepared by contacting the progestogen with a cellulosic binder. The binder may comprise, without limitation, one or more of methylcellulose, sodium carboxymethylcellulose (for example Tylose^{™}), ethylcellulose (for example Ethocel^{™}), hydroxypropyl methyl cellulose (HPMC) (Shin-Etsu Chemical Co., Ltd.), and hydroxypropyl cellulose (for example Klucel^{™}). For tablet formulations, binders impart sufficient cohesion to the powders to permit normal processing such as sizing, lubrication, compression, film coating, and packaging, but still permit the tablet to disintegrate and the composition to dissolve upon ingestion. In the present invention, the cellulosic binders also impart enhanced stability and/or recoverability to the progestogen active ingredient. Typically, the progestogen-containing formulations of the invention comprise one or more cellulosic binders in the range of about 2% to about 10%, of the total weight of the unit dosage form.

According to one embodiment of this invention, the cellulosic binder is hydroxypropyl cellulose having a molecular weight in the range from about 30,000 to about 1,500,000, preferably in the range from about 50,000 to about 150,000.

According to one embodiment of this invention, the cellulosic binder is hydroxypropyl cellulose, preferably hydroxypropyl cellulose fulfilling the requirements of European Pharmacopoeia and/or the United States National Formulary.

In one embodiment of the invention, the progestogen is NETA, the binder is Klucel^{™}, and the unit dosage form is a tablet containing about 0.1 to about 0.25 mg NETA and about 3.2 mg Klucel^{™} (total tablet weight = 80-85 mg).

The formulations of the invention may optionally comprise one or more diluents. Suitable diluents include, without limitation, either individually or in combination, lactose USP; lactose USP, anhydrous; lactose USP, spray dried; starch USP; directly compressible starch; mannitol USP; sorbitol; dextrose monohydrate; microcrystalline cellulose NF; dibasic calcium phosphate dihydrate NF; sucrose-based diluents; confectioner's sugar; monobasic calcium sulfate monohydrate; calcium sulfate dihydrate NF; calcium lactate trihydrate granular NF; dextrates NF (for example, Emdex^{™}); Celutab^{™}; dextrose (for example, Cerelose^{™}); inositol; hydrolyzed cereal solids such as the Maltrons^{™} and Mor-Rex^{™}; amylose; Rexcel^{™}; calcium carbonate; glycine; bentonite; and the like. The formulations typically comprise one or more diluents in the range of about 5% to about 99%, preferably about 25% to about 90%, and more preferably about 40% to about 80%, of the total weight of the composition.

The formulations of the invention may optionally comprise one or more disintegrants, particularly for tablet formulations. Suitable disintegrants include, without limitation, either individually or in combination, starches; sodium starch glycolate; clays (such as Veegum^{™}HV); alginates; pregelatinized corn starches (such as National^{™} 1551 and National^{™} 1550); and gums (such as agar, guar, locust bean, Karaya^{™}, pectin, and tragacanth). Disintegrants can be added at any suitable step during the preparation of the pharmaceutical composition, particularly prior to granulation or during the lubrication step prior to compression. The present pharmaceutical compositions comprise one or more disintegrants in the range of about 5% to about 99%, preferably about 25% to about 90%, and more preferably about 40% to about 80%, of the total weight of the composition.

The formulations of the invention optionally comprise one or more lubricants and/or glidants as a carrier material. Suitable lubricants and/or glidants include, without limitation, either individually or in combination, such lubricants and/or glidants as glyceryl behenate (Compritol^{™} 888); metalllic stearates (for example, magnesium, calcium and sodium stearates); stearic acid; hydrogenated vegetable oils (for example, Sterotex^{™}); talc; waxes; Stearowet^{™}; boric acid; sodium benzoate and sodium acetate; sodium chloride; DL-Leucine; polyethylene glycols (for example, Carbowax^{™} 4000 and Carbowax^{™} 6000); sodium oleate; sodium benzoate; sodium acetate; sodium lauryl sulfate; sodium stearyl fumarate (Pruv.TM.); and magnesium lauryl sulfate. The present pharmaceutical compositions comprise one or more lubricants at about 0.1% to about 10%, preferably about 0.2% to about 8%, and more preferably about 0.25% to about 5%, of the total weight of the composition. Magnesium stearate is a preferred lubricant used to reduce friction between the equipment and granulation during compression.

Talc is a preferred anti-adherent or glidant agent used to reduce sticking to equipment surfaces and also to reduce static in the blend. The compositions preferably comprise talc at about 0.1% to about 10%, more preferably about 0.25% to about 5%, and still more preferably about 0.5% to about 2%, of the total weight of the composition.

The formulations of the invention optionally comprise one or more wetting agents, particularly for tablet formulations. Suitable wetting agents include, either individually or in combination, such wetting agents as oleic acid; glyceryl monostearate; sorbitan monooleate; sorbitan monolaurate; triethanolamine oleate; polyoxyethylene sorbitan mono-oleate; polyoxyethylene sorbitan monolaurate; sodium oleate; and sodium lauryl sulfate. Wetting agents that are anionic surfactants are preferred. The formulations of the invention typically comprise one or more wetting agents present at about 0.1% to about 15%, preferably about 0.25% to about 10%, and more preferably about 0.5% to about 5%, of the total weight of the composition.

Other carrier materials (such as colorants, flavors and sweeteners) and modes of administration are known in the pharmaceutical art and can be used in the preparation of the formulations of the present invention.

In one preferred embodiment, the formulation according to this invention has a loss of mass (w/w) of not more than 15%, preferably of not more than 10 %, when tested according to European Pharmacopoeia 4th edition 2002, item 2.2.32, test C.

In another preferred embodiment, the formulation according to this invention disintegrates within 2 hours, preferably 1 hour, more preferred 30 minutes, most preferred 15 minutes when tested by the method described in European Pharmacopoeia 4th edition 2002, item 2.9.1, test A.

### Dosage forms and coatings

A dosage form, as used herein, refers to a formulation that is ready for administration to a subject. In practicing the present invention, progestogen-containing compositions may be formulated as tablets and the like. The invention also encompasses multilayered tablets wherein a given layer may represent a different drug. The tablet may also be scored to facilitate division of dosing. Alternatively, the dosage forms of the present invention may be unit dosage forms wherein the dosage form is intended to deliver one therapeutic dose per administration.

Preferably, the composition is formulated into a discrete dosage unit containing a predetermined amount of the active ingredients, such as tablets. Unit dosage tablets are preferred.

Coatings typically comprise one or more compounds that form a film, as well as one or more plasticizers.

Film-forming compounds include, without limitation, cellulosic compounds such as, for example, hydroxypropyl methyl cellulose, ethylcellulose, methacrylates, cellulose acetate phthalates, polyvinyl acetate phthalates, waxes, and silicone elastomers.

In a preferred embodiment, the cellulosic coating is selected from the group consisting of methylcellulose, sodium carboxymethylcellulose, preferably Tylose^{™}, ethylcellulose, preferably Ethocel^{™}, hydroxypropyl methyl cellulose (herein designated HPMC), and hydroxypropyl cellulose, preferably Klucel^{™}.

Plasticizers include, without limitation, glycerin, glycerol triacetate, propylene glycol, polyethylene glycol, triacetin, triethyl citrate, acetylated monoglycerides MACROGOL 6,000, and the like.

In one aspect, the invention provides progestogen-containing tablets coated with 1.5-5% w/w of a cellulosic film-forming compound relative to the total weight of the tablet, such as, for example, 1.5%, 2%, 2.5%, 3%, 3.5%, 4%, 4.5, and 5% w/w. In these embodiments, the progestogen content of the formulation is (i) not more than about 0.75%, 0.5%, 0.25%, or 0.1% w/w relative to the total formulation, such as, for example, between about 0.05-0.75%, 0.05-0.5%, 0.05-0.25%, 0.05-1.5%; 0.1-0.75%; 0.1-0.5%; 0.1-0.25%; 0.25-0.75%; 0.25-0.5% w/w, and the like, or (ii) less than about 0.35 mg/tablet, including, without limitation, not more than about 0.3, 0.25, 0.2, and 0.1 mg progestogen/tablet, such as, for example, between about 0.05-0.3 mg; 0.05-0.25 mg; 0.05-0.1 mg; 0.1-0.3 mg; 0.1-0.25 mg; and 0.1-0.2 mg per tablet.

In one embodiment, the formulation is a tablet containing 0.1-0.25 mg NETA and 3.2 mg Klucel^{™} (total tablet weight = 80-85 mg) and coated with 3% methylhydroxypropylcellulose E3 (total weight about 2.3 mg/tablet).

The formulations of the invention may be manufactured using conventional means of granulation and tabletting, including, without limitation, wet granulation and tabletting followed by film-coating or direct compression followed by film-coating.

Typically, the tablet process comprises the steps of preparing granules suitable for tableting by blending a mixture comprising the medicament, a binder, and optionally, a disintegrant and filler; adding a predetermined amount of water or granulation fluid to form a wet mass blend; sizing the wet mass blend into granules to aid drying; drying the wet granules to remove excess moisture; sizing the dried granules into granules suitable for tableting, and adding lubricant, one or more fillers, one or more dry binders, optionally a disintegrant, and other excipients necessary for tableting the granules; and a film-coating step.

Methods for preparing the formulations of the invention are well-known in the art and can be found, for example, in Remington, 19th Edition, Vol. II, Chapter 92, (Mack Publishing Company, Easton PA, 1995).

### Other active ingredients

In addition to progestogen, the formulations of the invention may comprise one or more additional active ingredients, including, without limitation, an estrogen, a second progestogen product, an androgenic agent, an androgen agonist, a progestogen agonist, an estrogen antagonist, or another hormone product. Estrogen, for example, may include, without limitation, ethinyl estradiol, mestranol, conjugated equine estrogen, estrone, estradiol, esterified estrogens, estropipate, and other estrogen equivalents and estrogen agonists.

In one embodiment, the formulation is a tablet containing 0.5 mg estradiol in addition to 0.1 or 0.25 mg NETA.

### Methods of stabilization

The present invention also provides methods for stabilizing a progestogen active ingredient in a pharmaceutical composition, particularly a dry composition such as, for example, a tablet, in which the progestogen is present in a proportion of not more than 1.5% w/w relative to the total weight of the composition, which are carried out by contacting the progestogen with a cellulosic binder such that the cellulosic binder comprises between about 0.5% to about 25%, such as, for example, about 0.75% to about 15% or about 1% to about 10%, of the total weight of the composition. Non-limiting examples of suitable cellulosic binders include methylcellulose, sodium carboxymethylcellulose (for example Tylose^{™}), ethylcellulose (for example Ethocel^{™}), hydroxypropyl methyl cellulose (HPMC) (Shin-Etsu Chemical Co., Ltd.), and hydroxypropyl cellulose (for example Klucel^{™}). However, it will be understood that any cellulosic binder may be used that imparts enhanced stability and/or recoverability to the formulation. Typically, use of a cellulosic binder in a dilute progestogen formulation according to the present invention results in an enhancement of at least about 5%, preferably about 10%, more preferably about 25%, and most preferably about 50%, in one or more parameters of stability/recoverability.

A typical parameter of stability/recoverability (as recommended by the International Conference on Harmonisation of Technical Requirements for Registration of Pharmaceuticals for Human Use) is shelf life, which is defined as the time period during which a drug product is expected to remain within the approved shelf life specification. The ICH guidelines provide that accelerated testing may be performed for a 6-month period at 40°C/75% relative humidity, and that a significant change in drug concentration is about 5% from the initial assay values under accelerated testing conditions. In one series of embodiments, the stability of the progestogen component is enhanced relative to the stability of progestogen in a parallel formulation in which the polyvinylpyrrolidone is replaced with an equivalent amount of hydroxypropylcellulose. In another series of embodiments, the stability of the progestogen component is enhanced relative to the stability of progestogen in a parallel formulation in which the gelatin is replaced with an equivalent amount of hydroxypropylcellulose. In these two last-mentioned tests, the stability of the progestogen component is enhanced at least 5%, preferably at least 10%, more preferred at least 15 %.

In another aspect, the present invention also provides methods for stabilizing a progestogen active ingredient in a pharmaceutical composition, particularly a dry composition such as, for example, a tablet, in which the progestogen is present in a proportion of not more than 1.5% w/w relative to the total weight of the composition, which methods are carried out by coating the progestogen-containing dosage form with 0.5-5% w/w of a cellulosic film-forming compound relative to the total weight of the tablet, such as, for example, 1.5%, 2%, 2.5%, 3%, 3.5%, 4%, 4.5, and 5% w/w.

### Methods of treatment

The references to methods of treatment in this description are to be interpreted as references to the compounds, pharmaceutical compositions and medicaments of the present invention for use in a method for treatment of the human (or animal) body by therapy (or for diagnosis).

The present disclosure also encompasses methods for treatment of progestogen-responsive syndromes, such as, for example, in hormone replacement therapy. Also encompassed are other modes of progestogen administration such as, for example, for contraception and treatment of osteoporosis. The methods are carried out by administering the formulation described above to a subject in need of a progestogen (or, a combination of a progestogen and another active ingredient, such as, for example, an estrogen). Progestogen-responsive syndromes include, without limitation, infertility related to non-receptive uterus, premenstrual tension, ovulation, primary dysmenorrhea and endometriosis, habitual abortion, respiratory depression in the Pickwickian syndrome, secondary amenorrhea, dysfunctional uterine bleeding, preeclampsia and toxemia of pregnancy, sexual infantilism, and post-menopausal symptoms.

The formulation of this invention is administered in a manner known per se, for example according to the instructions given by a doctor. A convenient way of administration is via the oral route (per orally).

The mentioning herein of a reference is no admission that it constitutes prior art.

All headings and sub-headings are used herein for convenience only and should not be construed as limiting the invention in any way.

Herein the word "comprise" is to be interpreted broadly meaning "include", "contain" or "comprehend" (vide, for example, Guidelines for Examination in the European Patent Office).

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### Example 1: Enhanced stability of NETA in a low-dose formulation

The purpose of this study was to compare the effect of (i) different binders and (ii) different coatings on stability of NETA is a low-dose formulation.

Table II below illustrates the test formulations:

**Table II**

| **Ingredients** | **Quantity (mg/tablet)** | **Function** | **Commercial Source** |
|---|---|---|---|
| **Active ingredients** | | | |
| Estradiol hemihydrate | 0.517 | Active substance | Schering AG, Germany / Diosynth B.V., Holland |
| Norethisterone Acetate | 0.100 | Active substance | Schering/Diosynth |

| **Other ingredients** | | | |
|---|---|---|---|
| Lactose monohydrate | 37.5 | Filler | DMV International, Holland |
| Maize starch | 37.5 | Disintegrant / filler | Cerestar Scandinavia |
| **Binder** | 3.20 | Binder | |
| Talc | 0.800 | Glidant / lubricant | Luzenac, Italy |
| Magnesium stearate | 0.400 | Lubricant | Acros Chemicals |

Two different binders were tested: Polyvidon^{™} VA 64 (polyvinylpyrollidone, BASF, Germany); and Klucel^{™} EF Pharm (hydroxypropyl cellulose, Aqualon, USA).

Tabletting and coating: Tablets containing the two binders were formed by fluid-bed granulation, compression on rotary press, film-coating in a coating pan using an air atomizing spray system. For film coating, either 1% or 3% Methocel E3 was used. The final tablets contained the film-coat components mentioned in tables III and IV, below:

**Table III**

| 1% film coat: | |
|---|---|
| **Film-coating: (theoretical quan-tity mq/tablet)** | 0.864 |
| Filmformer | 0.821 |
| Glycerol triacetate | 0.043 |

**Table IV**

| 3% film coat: | |
|---|---|
| **Film-coating: (theoretical quan-tity mq/tablet)** | 2.400 |
| Filmformer | 2.280 |
| Glycerol triacetate | 0.120 |

Packaging: Following coating, each tablet batch was packed in a calendar dial pack ("Dispenser").

Stability testing: The packaged products were stored at 40°C ± 2°C/ 75% relative humidity (hereinafter designated RH) ± 5% RH for 6 months. The products were subjected to assay analysis during the 6-months period. Statistically analysis was based upon analysis after 0, 3, and 6 months. At 6 months after initiation of the study, products were subjected to assay analysis using HPLC to detect NETA and estradiol.

### Results:

A statistical evaluation of up to 6-month assay data from storage condition 40°C/75 %RH was performed. Differences in formulations were evaluated by comparing the degradation rates (slopes) of NETA. NETA degradation was found to be dependent on both binder and film-coat amount. The type of film former (Methocel E3 or E5) did not significantly affect degradation.

The time in months until a significant change (5 %) at 40°C/75%RH (Ref. ICH Q1 A(R2) 'Stability Testing of New Drug Substances and Products') of NETA assay occurred was calculated based on the degradation rates.

Estimates of the equivalent degradation rates at 25°C were calculated using the Arrhenius equation in accordance with the principle in 'Chemical Stability of Pharmaceuticals. A Handbook for Pharmacists' Connor, K.A., Amidon, G. L. and Kennon, L. John Wiley & Sons, New York, 1978. The degradation rates were then used to calculate the time in months until a significant change of NETA occurred at 25°C.

The results are given in table V below:

**Table V**

| **Film-coat amount, %** | **Binder** | **The time (months) until a significant (5 % label claim) change in assay NETA occurs** | |
|---|---|---|---|
| | | **40°C** | **25°C** |
| 1 | Klucel EF | 3.5 | 18.3 |
| | Polyvidon VA64 | 3.2 | 16.6 |
| 3 | Klucel EF | 5.0 | 25.9 |
| | Polyvidon VA64 | 3.9 | 20.4 |

The results demonstrate that Klucel EF is superior to Polyvidon in enhancing NETA stability and that 3% film coat is superior to 1 %.

The results demonstrate that (i) cellulosic binders are superior to polyvidon in enhancing NETA stability and (ii) a 3% Methocel coat is superior to a 1% coat in enhancing NETA stability.

The disintegration time of the tablets, tested according to the European Pharmacopoeia 4th edition 2002, item 2.9.1. test A, was less than 10 minutes.

## Claims

1. A pharmaceutical tablet comprising
(i) norethindrone acetate (NETA) in the range from 0.05 to 0.35 mg per unit dosage form or the corresponding amount of norethindrone (NET) or a corresponding amount of a soluble ester or a salt of NET, and
(ii) a cellulosic binder in the range from 2 to 10% w/w, and
(iii) a cellulosic film-coating in the range 1.5 to 5% w/w.

2. The tablet according to claim 1, wherein said cellulosic binder is selected from the group consisting of methylcellulose, sodium carboxymethylcellulose, ethylcellulose, hydroxypropyl methyl cellulose herein designated HPMC, and hydroxypropyl cellulose.

3. The tablet according to any one of the preceding claims wherein said cellulosic binder is hydroxypropyl cellulose.

4. The tablet according to any one of the preceding claims wherein said cellulosic binder is hydroxypropyl cellulose having a molecular weight in the range from 30,000 to 1,500,000, preferably in the range from 50,000 to 150,000.

5. The tablet according to the preceding claims wherein the content of NETA is in the range from 0.07 to 0.13 mg per unit dosage.

6. The tablet according to any one of claims 1 to 4 wherein the content of NETA is in the range from 0.1 to 0.35 mg per unit dosage.

7. The tablet according to any one of the preceding claims further comprising lactose in an amount in the range from 30 to 45 mg, optionally as lactose monohydrate.

8. The tablet according to any one of the preceding claims further comprising maize starch in an amount in the range from 30 to 45 mg.

9. The tablet according to any one of the preceding claims comprising hydroxypropylcellulose in an amount in the range from 2 to 10 mg.

10. The tablet according to any one of the preceding claims comprising talc in an amount in the range from 0.5 to 2 mg.

11. The tablet according to any one of the preceding claims comprising magnesium stearate in an amount in the range from 0.1 to 1 mg.

12. The tablet according to any one of the preceding claims comprising hydroxypropylmethyl cellulose as film coater in an amount in the range from 0.5 to 3 mg.

13. The tablet according to any one of the preceding claims comprising glycerol triacetate in an amount in the range from 0.05 to 0.2 mg.

14. The tablet according to any one of claims 1 to 11 or 13 comprising an amount in the range from 0.2 to 1 mg of estradiol hemihydrate and an amount in the range from 2.5 to 4 mg of a cellulosic binder, wherein said cellulosic binder is selected from the group consisting of methylcellulose, sodium carboxymethylcellulose, ethylcellulose, hydroxypropyl methyl cellulose, and hydroxypropyl cellulose.

15. The tablet according to any one of the preceding claims comprising 0.5 mg of estradiol hemihydrate, 0.1 mg NETA, 40 mg of lactose monohydrate, 40 mg of maize starch, 3 mg of hydroxypropyl cellulose or hydroxypropylmethyl cellulose, 0.8 mg of talc, and 0.4 mg of magnesium stearate.

## Patentansprüche

1. Pharmazeutische Tablette, umfassend
(i) Norethindronacetat (NETA) im Bereich von 0,05 bis 0,35 mg pro Einheitsdosisform oder die entsprechende Menge an Norethindron (NET) oder eine entsprechende Menge eines löslichen Esters oder eines Salzes von NET, und
(ii) ein Cellulosebindemittel im Bereich von 2 bis 10 Gew.-%, und
(iii) eine Cellulosefilmbeschichtung im Bereich von 1,5 bis 5 Gew.-%.

2. Tablette nach Anspruch 1, wobei das Cellulosebindemittel ausgewählt ist aus der Gruppe bestehend aus Methylcellulose, Natriumcarboxymethylcellulose, Ethylcellulose, Hydroxypropylmethylcellulose, hierin als HPMC bezeichnet, und Hydroxypropylcellulose.

3. Tablette nach einem der vorhergehenden Ansprüche, wobei das Cellulosebindemittel Hydroxypropylcellulose ist.

4. Tablette nach einem der vorhergehenden Ansprüche, wobei das Cellulosebindemittel Hydroxypropylcellulose mit einem Molekulargewicht im Bereich von 30.000 bis 1.500.000, bevorzugt im Bereich von 50.000 bis 150.000 ist.

5. Tablette nach den vorhergehenden Ansprüchen, wobei der Gehalt an NETA im Bereich von 0,07 bis 0,13 mg pro Einheitsdosis liegt.

6. Tablette nach einem der Ansprüche 1 bis 4, wobei der Gehalt an NETA im Bereich von 0,1 bis 0,35 mg pro Einheitsdosis liegt.

7. Tablette nach einem der vorhergehenden Ansprüche, ferner umfassend Lactose in einer Menge im Bereich von 30 bis 45 mg, gegebenenfalls als Lactose-Monohydrat.

8. Tablette nach einem der vorhergehenden Ansprüche, ferner umfassend Maisstärke in einer Menge im Bereich von 30 bis 45 mg.

9. Tablette nach einem der vorhergehenden Ansprüche, umfassend Hydroxypropylcellulose in einer Menge im Bereich von 2 bis 10 mg.

10. Tablette nach einem der vorhergehenden Ansprüche, umfassend Talkum in einer Menge im Bereich von 0,5 bis 2 mg.

11. Tablette nach einem der vorhergehenden Ansprüche, umfassend Magnesiumstearat in einer Menge im Bereich von 0,1 bis 1 mg.

12. Tablette nach einem der vorhergehenden Ansprüche, umfassend Hydroxypropylmethylcellulose als Filmüberzug in einer Menge im Bereich von 0,5 bis 3 mg.

13. Tablette nach einem der vorhergehenden Ansprüche, umfassend Glycerintriacetat in einer Menge im Bereich von 0,05 bis 0,2 mg.

14. Tablette nach einem der Ansprüche 1 bis 11 oder 13, umfassend eine Menge im Bereich von 0,2 bis 1 mg Östradiolhalbhydrat und eine Menge im Bereich von 2,5 bis 4 mg eines Cellulosebindemittels, wobei das Cellulosebindemittel ausgewählt ist aus der Gruppe bestehend aus Methylcellulose, Natriumcarboxymethylcellulose, Ethylcellulose, Hydroxypropylmethylcellulose und Hydroxypropylcellulose.

15. Tablette nach einem der vorhergehenden Ansprüche, umfassend 0,5 mg Östradiolhalbhydrat, 0,1 mg NETA, 40 mg Lactose-Monohydrat, 40 mg Maisstärke, 3 mg Hydroxypropylcellulose oder Hydroxypropylmethylcellulose, 0,8 mg Talkum und 0,4 mg Magnesiumstearat.

## Revendications

1. Comprimé pharmaceutique comprenant
(i) de l'acétate de noréthindrone (NETA) dans la plage de 0,05 à 0,35 mg par forme posologique unitaire ou la quantité correspondante de noréthindrone (NET) ou une quantité correspondante d'un ester soluble ou d'un sel de NET, et
(ii) un liant cellulosique dans la plage de 2 à 10 % p/p, et
(iii) un enrobage pelliculaire cellulosique dans la plage de 1,5 à 5 % p/p.

2. Comprimé selon la revendication 1, dans lequel ledit liant cellulosique est choisi dans le groupe constitué de la méthylcellulose, la carboxyméthylcellulose de sodium, l'éthylcellulose, l'hydroxypropylméthylcellulose (ci-après HPMC) et l'hydroxypropylcellulose.

3. Comprimé selon l'une quelconque des revendications précédentes, dans lequel ledit liant cellulosique est l'hydroxypropylcellulose.

4. Comprimé selon l'une quelconque des revendications précédentes, dans lequel ledit liant cellulosique est de l'hydroxypropylcellulose ayant un poids moléculaire situé dans la plage de 30 000 à 1 500 000, de préférence dans la plage de 50 000 à 150 000.

5. Comprimé selon les revendications précédentes, dans lequel la teneur en NETA se situe dans la plage de 0,07 à 0,13 mg par dose unitaire.

6. Comprimé selon l'une quelconque des revendications 1 à 4, dans lequel la teneur en NETA se situe dans la plage de 0,1 à 0,35 mg par dose unitaire.

7. Comprimé selon l'une quelconque des revendications précédentes, comprenant en outre du lactose en une quantité située dans la plage de 30 à 45 mg, éventuellement sous forme de lactose monohydraté.

8. Comprimé selon l'une quelconque des revendications précédentes, comprenant en outre de l'amidon de maïs en une quantité située dans la plage de 30 à 45 mg.

9. Comprimé selon l'une quelconque des revendications précédentes, comprenant de l'hydroxypropylcellulose en une quantité située dans la plage de 2 à 10 mg.

10. Comprimé selon l'une quelconque des revendications précédentes, comprenant du talc en une quantité située dans la plage de 0,5 à 2 mg.

11. Comprimé selon l'une quelconque des revendications précédentes, comprenant du stéarate de magnésium en une quantité située dans la plage de 0,1 à 1 mg.

12. Comprimé selon l'une quelconque des revendications précédentes, comprenant de l'hydroxypropylméthylcellulose comme agent d'enrobage pelliculaire en une quantité située dans la plage de 0,5 à 3 mg.

13. Comprimé selon l'une quelconque des revendications précédentes, comprenant du triacétate de glycérol en une quantité située dans la plage de 0,05 à 0,2 mg.

14. Comprimé selon l'une quelconque des revendications 1 à 11 ou 13, comprenant une quantité située dans la plage de 0,2 à 1 mg d'hémihydrate d'estradiol et une quantité située dans la plage de 2,5 à 4 mg d'un liant cellulosique, ledit liant cellulosique étant choisi dans le groupe constitué de la méthylcellulose, la carboxyméthylcellulose de sodium, l'éthylcellulose, l'hydroxypropylméthylcellulose et l'hydroxypropylcellulose.

15. Comprimé selon l'une quelconque des revendications précédentes, comprenant 0,5 mg d'hémihydrate d'estradiol, 0,1 mg de NETA, 40 mg de lactose monohydraté, 40 mg d'amidon de maïs, 3 mg d'hydroxypropylcellulose ou d'hydroxypropylméthylcellulose, 0,8 mg de talc et 0,4 mg de stéarate de magnésium.
